# EUROPÄISCHE PATENTSCHRIFT

(11) **EP 4 146 107 B1**
(45) Veröffentlichungstag und Bekanntmachung des Hinweises auf die Patenterteilung: **23.08.2023**
(21) Anmeldenummer: 21724289.0
(22) Anmeldetag: 07.05.2021
(51) Int. Cl.: A61B 18/14, A61B 18/00

(54) **STEUERUNGSVORRICHTUNG UND ELEKTROCHIRURGISCHES INSTRUMENT**
CONTROL DEVICE AND ELECTROSURGICAL INSTRUMENT
DISPOSITIF DE COMMANDE ET INSTRUMENT ÉLECTRO-CHIRURGICAL

(30) Priorität: 07.05.2020 DE 102020112416
(43) Veröffentlichungstag der Anmeldung: 15.03.2023
(73) Patentinhaber: KLS Martin GmbH + Co. KG, 79108 Freiburg (DE)
(72) Erfinder: MARTIN, Lukas, 79100 Freiburg (DE); HERR, Sebastian, 79102 Freiburg i. Breisgau (DE)
(74) Vertreter: Mertzlufft-Paufler, Cornelius
(86) Internationale Anmeldenummer: PCT/EP2021/062225
(87) Internationale Veröffentlichungsnummer: WO 2021/224495

(56) Entgegenhaltungen:
- US-A1- 2007 043 352
- US-A1- 2007 282 333
- US-A1- 2014 276 736
- US-A1- 2016 175 022

## Beschreibung

Die Erfindung betrifft eine Steuerungsvorrichtung für ein elektrochirurgisches Instrument und ein elektrochirurgisches Instrument, insbesondere ein Hochfrequenz-Versiegelungsinstrument. Besonders bevorzugt kann es sich bei dem elektrochirurgischen Instrument um ein bipolares elektrochirurgisches Instrument handeln.

Steuervorrichtungen für elektrochirurgische Instrumente können wenigstens ein Schaltelement zum Aktivieren eines Stromflusses aufweisen.

Der Stromfluss kann dabei je nach Anwendung zum Beispiel zum Koagulieren oder zur Gefäßversiegelung während eines operativen Eingriffs genutzt werden.

Beim sogenannten bipolaren Koagulieren erfolgt eine Denaturierung des Gewebes, um eine Hämostase zu bewirken. Hierbei wird hochfrequenter Wechselstrom (folgend auch HF-Strom genannt) genutzt, um einen thermischen Effekt im Gewebe zu erzielen. Durch den spezifischen Widerstand wandelt sich die elektrische Energie beim stromdurchflossenen Gewebe in thermische Energie um und erhitzt so das Gewebe. Häufig wird die bipolare Koagulation durchgeführt um diffus auftretende Blutungen zu stillen. Bisher wird meist mit bipolaren Pinzetten koaguliert. Das Gewebe wird gefasst und/oder die Pinzettenspitzen auf die blutende Oberfläche gedrückt und mit Aktivierung über einen Fuß- oder Fingertaster das Gewebe zur Hämostase koaguliert.

Bei der sogenannten bipolaren Gefäßversiegelung werden unter Anderem Gefäße, aber auch Gewebeschichten versiegelt. Die Gefäßversiegelung ist eine Sonderform der Koagulation.

Zusätzlicher mechanischer Druck durch die gegenüberliegenden Elektroden bewirkt eine Neuausrichtung von Kollagen und Elastin, wodurch die Gefäßwände miteinander "verschmelzen" und abgedichtet werden. Instrumente hierfür sind meist bipolare Klemmen (offen chirurgisch und/oder laparoskopisch).

Man kennt bereits Steuerungsvorrichtungen, die ein feststehendes Griffteil und ein bewegliches Griffteil und ein Aktivierungselement zum Aktivieren eines Stromflusses umfassen, wobei das Aktivierungselement bei einer Betätigung des beweglichen Griffteils aus einer Deaktivierungsstellung in wenigstens eine Aktivierungsstellung verstellbar ist oder verstellt wird. Dies hat den Vorteil, dass ein Nutzer nicht neben der Kraftaufbringung für die Verstellung der Griffteile, zusätzlich und zeitgleich den Stromfluss unabhängig aktivieren muss, so dass ein vereinfachtes Arbeiten möglich ist. Vorbekannte Steuerungsvorrichtungen elektrochirurgischer Instrumente weisen hier jedoch Nachteile in der Handhabung auf.

Weiterhin sind vorbekannte Instrumente in der Regel auf nur ein Anwendungsgebiet abgestimmt, so dass es erforderlich sein kann, dass während einer Operation ein Chirurg einen Instrumentenwechsel vornehmen muss. Ein Instrumentenwechsel ist nicht nur für den Anwender unvorteilhaft aufgrund des damit verbundenen umständlichen Handlings der verschiedenen Instrumente, sondern der Instrumentenwechsel verlängert auch die OP-Zeit insgesamt. Insbesondere in Situationen, in denen schnelles Handeln vom Anwender gefordert ist, kann ein erforderlicher Instrumentenwechsel sogar bedrohlich für einen operierten Patienten sein, wenn z.B. eine auftretende Blutung schnell gestoppt werden müsste.

US 2014/0276736 A1 offenbart eine Vorrichtung zum Bearbeiten von Gewebe. Ein Endeffektor umfasst eine erste Backe, eine zweite Backe, einen Schaft und ein Verriegelungselement. Die zweite Backe kann relativ zu der ersten Backe von einer offenen Position in eine geschlossene Position verschwenken. Der Schaft hat ein scharfes distales Ende und verschiebt sich zwischen der ersten und der zweiten Backe. Der Schaft verschiebt sich von einer proximalen Position in eine erste distale Position, um die zweite Backe in die geschlossene Position zu verschwenken. Mit dem Endeffektor kann bipolare HF-Energie angewendet werden, wenn sich der Schaft in der ersten distalen Position befindet. Der Schaft verschiebt sich dann in eine zweite distale Position, um zwischen der ersten und der zweiten Backe eingeklemmtes Gewebe abzutrennen. Das Verriegelungselement verhindert, dass der Schaft von der ersten distalen Position in die zweite distale Position vorrückt, bis das Verriegelungselement betätigt wird.

US 2016/175022 A1 offenbart eine Vorrichtung zum Operieren von Gewebe. Die Vorrichtung umfasst einen Endeffektor, der am distalen Ende eines länglichen Schafts angeordnet ist und zwei bewegliche Backen umfasst, mit denen Gewebe gegriffen werden kann. Ein Auslöser ist konfiguriert, um die zweite Backe auf die erste Backe zu und von dieser weg zu bewegen. Der Verriegelungsmechanismus ist im verriegelten Zustand so konfiguriert, dass er eine Bewegung der zweiten Backe entlang eines ersten Pfads ermöglicht, wobei ein relativer Winkel zwischen der ersten Backe und der zweiten Backe auf einen ersten Maximalwinkel begrenzt ist. Der Verriegelungsmechanismus ist im entriegelten Zustand so konfiguriert, eine Bewegung der zweiten Backe entlang eines zweiten Pfads zu ermöglichen, wobei der relative Winkel zwischen der ersten Backe und der zweiten Backe auf einen zweiten Maximalwinkel begrenzt ist. Der zweite Maximalwinkel ist größer als der erste Maximalwinkel.

US 2007/043352 offenbart eine Vorrichtung zum operieren von Gewebe, insbesondere eine Versiegelungsvorrichtung. Die Vorrichtung umfasst ein feststehendes Griffteil, ein bewegliches Griffteil und ein Aktivierungselement zum Aktivieren eines Stromflusses, wobei das Aktivierungselement um einen Drehpunkt verstellt wird.

Es besteht daher die Aufgabe, eine Steuerungsvorrichtung für ein elektrochirurgisches Instrument eingangs genannter Art und/oder ein elektrochirurgisches Instrument mit jeweils verbesserten Gebrauchseigenschaften zu schaffen.

Die genannte Aufgabe wird erfindungsgemäß durch eine Steuervorrichtung mit den Merkmalen nach Anspruch 1 gelöst Bevorzugte Ausführungsformen sind in den abhängigen Ansprüchen offenbart.

Insbesondere wird erfindungsgemäß zur Lösung der Aufgabe eine Steuerungsvorrichtung eingangs genannter Art vorgeschlagen, wobei bei einer Betätigung des beweglichen Griffteils das Aktivierungselement um einen Drehpunkt, insbesondere um eine Drehachse, verstellbar ist oder verstellt wird und wobei das Aktivierungselement so ausgebildet ist, dass ein durch das Aktivierungselement aktivierter Strom durch das Aktivierungselement fließt oder fließen kann. Bei dem Strom kann es sich insbesondere um einen Versiegelungsstrom, beispielsweise den bereits zuvor erwähnten Versiegelungsstrom handeln. Somit ist eine besonders zuverlässige und leichtgängige Aktivierung möglich. Vorzugsweise kann dadurch ein Aktivieren eines Stromflusses eines ersten Typs, vorzugsweise eines Versiegelungsstroms vorgesehen sein.

Weiterhin wird insbesondere zur Lösung der Aufgabe eine Steuerungsvorrichtung für ein elektrochirurgisches Instrument vorgeschlagen, umfassend wenigstens ein feststehendes Griffteil, ein bewegliches Griffteil, wenigstens ein Schaltelement zum Aktivieren eines Stromflusses, wobei das Schaltelement durch einen Benutzer unabhängig von einer Betätigung des beweglichen Griffteils betätigbar ist, wobei sich die Steuerungsvorrichtung dadurch kennzeichnet, dass das Schaltelement am beweglichen Griffteil ausgebildet ist. Somit ist es möglich, dass ein Anwender auf einfache Weise eine Aktivierung eines Stromflusses vornehmen kann, unabhängig davon, ob ein mechanischer Druck durch Betätigung des beweglichen Griffteils auf Gewebe aufgebracht wird oder nicht. Beispielsweise kann somit ein Aktivieren eines Stromflusses eines zweiten Typs, vorzugsweise eines Koagulationsstroms und/oder eines Schneidestroms, ermöglicht werden. Das wenigstens eine Schaltelement kann zum Beispiel als ein Taster ausgebildet sein. Vorzugsweise kann das wenigstens eine Schaltelement als ein Drucktaster ausgestaltet sein, der insbesondere ein haptisches Feedback bei seiner Aktivierung liefert. Gemäß einer Weiterbildung ist es möglich, dass die Steuerungsvorrichtung zwei oder mehr als zwei Schaltelemente, insbesondere zur Aktivierung und/oder zum Umschalten zwischen wenigstens zwei Bipolarströmen aufweist.

Kombiniert man somit die beiden zuvor beschriebenen Ausgestaltungen können mehrere Anwendungsgebiete durch eine Steuerungsvorrichtung und/oder ein elektrochirurgisches Instrument abgedeckt werden, so dass kein Instrumentenwechsel erforderlich ist. Über das wenigstens eine Schaltelement kann der Anwender daher also unabhängig von einer Maulteilöffnung des elektrochirurgischen Instruments ebenfalls HF-Strom aktivieren, welcher zum bipolaren Koagulieren von Gewebe genutzt wird. Das wenigstens eine Schaltelement kann insbesondere ein vom bipolaren Versiegeln differenzierbares Schaltsignal aktivieren, wodurch dieses im Generator erkannt wird und der entsprechende HF-Strom fließt. Dadurch, dass die Maulteile auch im geöffneten Zustand aktiviert werden können, kann der Anwender die Maulteile (bzw. das Instrument) äquivalent zu einer bipolaren Pinzette nutzen.

Insbesondere wird somit weiter erfindungsgemäß zur Lösung der Aufgabe eine Steuerungsvorrichtung für ein elektrochirurgisches Instrument, umfassend wenigstens ein Schaltelement zum Aktivieren und/oder Umschalten eines bipolaren Stromflusses vorgeschlagen, das dadurch gekennzeichnet ist, dass durch das wenigstens eine Schaltelement ein Aktivieren und/oder ein Umschalten zwischen wenigstens zwei unterschiedlichen Schaltsignalen möglich ist, um durch einen Generator des elektrochirurgischen Instruments in Abhängigkeit vom jeweiligen Schaltsignal wenigstens zwei unterschiedliche Bipolarströme zu erzeugen. Insbesondere kann dabei vorgesehen sein, dass durch die wenigstens zwei Schaltsignale wenigstens ein bipolarer Koagulationsstrom und/oder Schneidestrom und ein bipolarer Versiegelungsstroms aktivierbar sind. Abgesehen vom mechanischen Druck auf das Gewebe sind auch die Stromformen des Generators eines elektrochirurgischen Instruments maßgebend für die unterschiedlichen Applikationen (wie z.B. bipolares Koagulieren und Versiegeln), wodurch eine Differenzierbarkeit des Schaltsignals beider Anwendungen im Instrument nötig wird. Die beiden Schaltsignale können beispielsweise durch entgegengesetzte Dioden am Schalter/Taster eindeutig differenziert werden. Der Generator erkennt die unterschiedlichen Aktivierungszustände und gibt dementsprechend verschiedene Ströme ab. Insbesondere kann der Strom bei der bipolaren Gefäßversiegelung generatorseitig geregelt und beendet und/oder der Strom der bipolaren Koagulation über den Anwender aktiviert und beendet werden.

Ein Anwender muss daher während einer Operation keinen Instrumentenwechsel mehr durchführen. Dadurch vereinfacht sich der Operationsvorgang für ihn erheblich. Zudem kann dadurch eine Zeitersparnis gegenüber Operationen erreicht werden, bei welchen ein Instrumentenwechsel erforderlich ist.

Beispielsweise kann es vorgesehen sein, dass das wenigstens eine Steuerelement durch einen Nutzer ein unmittelbar betätigbares und/oder sprachsteuerbares Schaltelement ist. Das wenigstens eine Schaltelement kann also von einem Nutzer durch einen über einen Finger und/oder eine Hand aufgebrachte Kraft betätigt werden. Alternativ oder ergänzend dazu kann es über definierbare oder vordefinierte Sprachbefehle gesteuert werden, also insbesondere ein Umschalten und/oder ein Einschalten und/oder ein Abschalten eines HF-Stromes.

Nachfolgend werden weitere vorteilhafte Ausgestaltungen der Erfindung beschrieben, die allein oder in Kombination mit den Merkmalen anderer Ausgestaltungen optional zusammen mit den Merkmalen aus Anspruch 1 und/oder Anspruch 2 und/oder Anspruch 3 kombiniert werden können.

Gemäß einer vorteilhaften Weiterbildung, kann das Aktivierungselement als eine Schaltwippe ausgebildet sein. Somit ist eine besonders verschleiß- und wartungsarme Ausgestaltung möglich.

Gemäß einer vorteilhaften Weiterbildung kann es vorgesehen sein, dass ein Aktivierungselement, beispielsweise das bereits zuvor genannte Aktivierungselement, am beweglichen Griffteil und/oder am feststehenden Griffteil ausgebildet oder angeordnet ist. Insbesondere kann eine Drehachse des Aktivierungselements, beispielsweise die bereits zuvor genannte Drehachse des Aktivierungselements, am beweglichen Griffteil ausgebildet sein. Somit ist es möglich, die Verstellung des Aktivierungselements bei einer Betätigung des beweglichen Griffteils relativ zum feststehenden Griffteil auf einfache Weise erreichen zu können.

Um einen Stromfluss durch Schließen eines Stromkreises anhand des Aktivierungselements vornehmen zu können, kann gemäß einer vorteilhaften Weiterbildung vorgesehen sein, dass das Aktivierungselement wenigstens ein Kontaktelement aufweist und dass die Steuerungsvorrichtung eine Gegenkontaktfläche aufweist, die in einer Aktivierungsstellung durch das Kontaktelement beaufschlagt ist. Bevorzugt ist hierbei vorgesehen, dass das Kontaktelement bei einer Verstellung des beweglichen Griffteils über einen Aktivierungsabschnitt eines Gesamtverstellweges des beweglichen Griffteils und/oder solange das Aktivierungselement in einer Aktivierungsstellung ist, entlang der Gegenkontaktfläche gleitend geführt ist. Somit kann eine Gefäßversiegelung nur durch das Verstellen des beweglichen Griffteils relativ zum feststehenden Griffteil erreicht werden, wobei dabei zunächst eine mechanische Kraftübertragung beispielsweise durch zwei sich gegenüberliegende und/oder verschließbare Branchen (auch Maulteile genannt), welche zum Beispiel an einem distalen Ende eines Endeffektors angebracht sind und zum Greifen des zu manipulierenden Gewebes vorgesehen sind, aufgebracht wird. Über die Griffteilstellung (und optional einer gekoppelten Feder) wird ein erzeugter Druck auf das zwischen den Maulteilen befindliche Gewebe gesteuert. Bei einer bestimmten, definierten Griffteilstellung und insbesondere einem damit erzeugten Druck auf dem Gewebe, wird das Aktivierungselement also in die Aktivierungsstellung gebracht, was wiederum den HF-Strom zwischen den Maulteilen aktiviert.

Dabei kann es vorgesehen sein, dass in allen Aktivierungsstellungen des Aktivierungsbereichs des Griffteiles der Kontakt des Aktivierungselements über das Kontaktelement mit dem Gegenkontaktelement erhalten bleibt, wodurch der HF-Strom aktiviert bleibt, bis zum Beispiel entweder über einen Generator und/oder einen Regelalgorithmus der Versiegelungsprozess als beendet erkannt wird, oder bis der Anwender die Griffteile des Griffs öffnet und das Aktivierungselement deaktiviert wird, also bis kein Kontakt mehr zwischen dem Kontaktelement und dem Gegenkontaktelement vorliegt. Da die Aktivierung des HF-Stroms über die Hebelstellung des Griffs und damit dem Druck zum Beispiel zwischen zwei Maulteilen gesteuert ist, ist ein ausreichender Prozessdruck der Branchen bei der Gefäßversiegelung gegeben.

Um einen Stromfluss durch Schließen eines Stromkreises anhand des Aktivierungselements vornehmen zu können, kann vorgesehen sein, dass das Kontaktelement und die Gegenkontaktfläche leitend sind. Das Kontaktelement kann insbesondere eine leitende Kontaktfläche aufweisen. Die Steuerungsvorrichtung kann daher so ausgebildet sein, dass durch eine Kontaktierung des leitenden Kontaktelements mit der leitenden Gegenkontaktfläche ein zunächst offener Stromkreis geschlossen werden kann.

Hierzu ist bevorzugt vorgesehen, dass das Aktivierungselement mindestens ein weiteres leitendes Kontaktelement, beispielsweise mit einer leitenden Kontaktfläche, hat und dass die mindestens zwei leitenden Kontaktelemente miteinander leitend verbunden sind. Das weitere leitende Kontaktelement ist bevorzugt permanent mit einem leitenden Gegenkontakt elektrisch verbunden. Es kann allerdings auch vorgesehen sein, dass der Gegenkontakt eine Gegenkontaktfläche aufweist, mit welchem das weitere leitende Kontaktelement bei der Verstellung des beweglichen Griffteils in Kontakt kommt.

Bevorzugt ist das Aktivierungselement ein leitendes Bauteil, insbesondere eine leitende Schaltwippe.

Das Aktivierungselement kann insbesondere aus Metall sein.

Gemäß einer vorteilhaften Weiterbildung kann die Steuerungsvorrichtung wenigstens ein Rückstellelement aufweisen, das derart eingerichtet und angeordnet ist, dass bei einer Betätigung des beweglichen Griffteils das Aktivierungselement entgegen einer Rückstellkraft des wenigstens einen Rückstellelements aus einer Ruheposition auslenkbar ist oder ausgelenkt wird. Insbesondere kann das Rückstellelement als wenigstens ein Federelement und/oder als wenigstens ein Magnet ausgebildet sein. Vorzugsweise weist die Steuerungsvorrichtung wenigstens zwei mit dem Aktivierungselement gekoppelte oder koppelbare Rückstellelemente auf, insbesondere zwei, vorzugsweise entgegen einander wirkende, Rückstellelemente. Weiter bevorzugt kann dabei vorgesehen sein, dass in der Ruheposition keine Krafteinwirkung des wenigstens einen Rückstellelements oder sich gegenseitig neutralisierende Krafteinwirkungen der Rückstellelemente auf das Aktivierungselement wirken.

Gemäß einer weiteren vorteilhaften Weiterbildung kann die Steuerungsvorrichtung ein Führungselement aus einem nicht elektrisch leitenden Material aufweisen, wobei bei einer Verstellung des beweglichen Griffteils aus einer maximalen Offenstellung in Richtung einer Schließstellung wenigstens ein Kontaktelement, beispielsweise das bereits zuvor genannte wenigstens eine Kontaktelement des Aktivierungselements über einen Deaktivierungsabschnitt, insbesondere einen ersten Deaktivierungsabschnitt, eines oder des Gesamtverstellweges des Kontaktelements entlang einer ersten Führungsfläche geführt sein kann. Insbesondere kann das Aktivierungselement am Führungselement aus seiner Ruheposition um einen Winkel auslenkbar sein oder ausgelenkt werden. Alternativ oder ergänzend zu einem isolierenden Führungselement kann gemäß einer weiteren vorteilhaften Weiterbildung vorgesehen sein, dass ein Luftraum als Isolator genutzt wird. Somit kann das Kontaktelement zumindest über einen Teilbereich des Deaktivierungsabschnitts innerhalb des Luftraums liegen, so dass kein Stromfluss möglich ist.

Um einem Nutzer eine Rückmeldung zur Schaltstellung während einer Bedienung der Steuerungsvorrichtung geben zu können, kann gemäß einer vorteilhaften Weiterbildung vorgesehen sein, dass die Steuerungsvorrichtung wenigstens ein Feedbackelement aufweist, mit welchem dem Nutzer bei einer Bewegung des beweglichen Griffteils eine haptische und/oder eine akustische Rückmeldung vermittelbar ist. Insbesondere kann dem Nutzer dadurch eine Rückmeldung vermittelt werden, wenn eine Verstellung des Aktivierungselements zwischen einer Deaktivierungsstellung und einer Aktivierungsstellung erfolgt. Somit kann der Nutzer aufgrund der Rückmeldung daraus schließen, ob ein Stromfluss aktiviert ist oder nicht oder ob eine Aktivierung und/oder Deaktivierung kurz bevor steht, ohne dass er seinen Blick von einer Operationsstelle auf eine Anzeige oder dergleichen abwenden muss. Vorzugsweise kann die Steuerungsvorrichtung ein Führungselement, beispielsweise das bereits zuvor genannte Führungselement aufweisen, wobei das Führungselement in einer ersten Führungsfläche eine Richtungsänderung, insbesondere eine Mulde und/oder eine Erhöhung, aufweist, durch welche dem Benutzer eine haptische Rückmeldung vermittelbar ist. Durch das Feedbackelement kann somit ein zu überwindender Widerstand eingerichtet sein, der überwunden werden muss, um in eine andere Schaltposition (z.B. aktiviert oder deaktiviert) zu gelangen.

Gemäß einer vorteilhaften Ausgestaltung der Steuerungsvorrichtung kann eine Gegenkontaktfläche, insbesondere die zuvor genannte Gegenkontaktfläche, fest mit einem Trägerelement verbunden sein. Alternativ oder ergänzend dazu kann das Trägerelement fest mit dem feststehenden Griffteil verbunden sein.

Nach einer weiteren vorteilhaften Ausgestaltung der Steuerungsvorrichtung kann die Gegenkontaktfläche an einem steifen Gegenkontaktelement ausgebildet sein, insbesondere wobei die Gegenkontaktfläche wenigstens eine Richtungsänderung, vorzugsweise eine Kurve, aufweist.

Gemäß einer vorteilhaften Ausführung der Steuerungsvorrichtung kann eine Gegenkontaktfläche, insbesondere die zuvor genannte Gegenkontaktfläche, an einem wenigstens teilweise verbiegbaren Gegenkontaktelement ausgebildet sein. Insbesondere kann das Gegenkontaktelement als federndes Blech ausgebildet sein.

In beiden Fällen kann das Kontaktelement durch die Gegenkontaktfläche während einer Aktivierungsstellung geführt sein und/oder die Gegenkontaktfläche beaufschlagen, um einen Stromkreis zu schließen.

Gemäß einer weiteren Ausgestaltung kann vor dem Gegenkontaktelement ein oder das bereits genannte, vorzugsweise elektrisch isolierende, Feedbackelement angeordnet sein, durch welches dem Benutzer eine akustische und/oder haptische Rückmeldung vermittelbar ist. Insbesondere kann das Feedbackelement durch das Aktivierungselement verbiegbar und/oder verdrängbar ausgestaltet sein, vorzugsweise wobei das Feedbackelement als ein federndes Blech ausgebildet ist.

Um die Zeitpunkte des Stromflusses auf eine bestimmte Stellung der Griffteile relativ zueinander einstellen zu können, kann die Steuervorrichtung eine Kulissenführung für das zumindest eine Kontaktelement des Aktivierungselements aufweisen, die dazu eingerichtet ist, dass bei einer Verstellung des beweglichen Griffteils relativ zum feststehenden Griffteil das Kontaktelement über einen ersten Deaktivierungsabschnitt des Gesamtverstellweges verstellt wird, dass dem ersten Deaktivierungsabschnitt in gleicher Verstellrichtung ein Aktivierungsabschnitt, insbesondere mit einem Endanschlag des Gesamtstellweges, nachgelagert ist, und dass bei einer entgegengesetzten Rückverstellung des beweglichen Griffteils relativ zum feststehenden Griffteil das Kontaktelement aus dem Aktivierungsabschnitt in einen zweiten Deaktivierungsabschnitt und/oder eine Ruheposition verstellt wird.

Gemäß einer weiteren vorteilhaften Ausgestaltung kann die Steuerungsvorrichtung zwischen dem beweglichen Griffteil und dem feststehenden Griffteil eine Sicherheitseinrichtung aufweisen, die eine Aktivierung eines Stromflusses verhindert, solange das bewegliche Griffteil relativ zum feststehenden Griffteil nicht über einen Endpunkt, insbesondere einen haptisch und/oder akustisch durch einen Benutzer wahrnehmbaren Endpunkt, eines Sicherheitsabschnitt des Gesamtverstellweges hinaus verstellt wurde, vorzugsweise wobei der Sicherheitsabschnitt kleiner als der erste Deaktivierungsabschnitt des Gesamtverstellweges ausgebildet ist.

Um eine Signalübertragung wenigstens eines auf dem beweglichen Griffteil angeordneten Schaltelements relativ verschleißfrei zu ermöglichen und/oder um eine Aktivierung eines Stromflusses erst nach einer teilweisen Betätigung des beweglichen Griffteils zu ermöglichen, kann eine Sicherheitseinrichtung zwischen dem beweglichen Griffteil und dem feststehenden Griffteil ein federndes Kontaktmittel, insbesondere einen federnd gelagerten Hubstift, und ein Gegenkontaktmittel aufweisen. Vorzugsweise wobei ab Erreichen eines Endpunktes eines Sicherheitsabschnitts ein Kontaktschluss zwischen dem Kontaktmittel und dem Gegenkontaktmittel vorliegt und/oder wobei bei einer fortgesetzten Verstellung bis zu einem Endanschlag des Gesamtstellweges der Kontaktschluss bestehen bleibt.

Gemäß einer bevorzugten Weiterbildung kann wenigstens ein Schaltelement oder das wenigstens eine Schaltelement an dem beweglichen und/oder an dem feststehenden Griffteil und/oder an einem Fußschaltelement ausgebildet sein. Dies hat den Vorteil, dass es durch einen Nutzer unabhängig von der Stellung der Griffteile stets einfach erreichbar und betätigbar ist.

Gemäß einer besonders bevorzugten Ausgestaltung kann das bewegliche Griffteil und das Aktivierungselement unterschiedliche, insbesondere räumlich beabstandet angeordnete, Drehachsen aufweisen. Somit ist eine größere relative Auslenkung des Aktivierungselements selbst bei geringer Verstellstrecke des Griffteils möglich.

Gemäß einer weiteren vorteilhaften Ausgestaltung können die innenliegend verbauten Bauteile, insbesondere die innenliegenden elektrischen Bauteile, wenigstens des beweglichen Griffteils zumindest teilweise innerhalb eines versiegelten Innenraums, insbesondere luft- und/oder wasserdicht versiegelten Innenraums, eingehaust sein, vorzugsweise wobei der Innenraum durch Umspritzen eines Vorspritzlings ausgebildet ist.

Die Erfindung betrifft weiter ein elektrochirurgisches Instrument, insbesondere ein Hochfrequenz-Versiegelungsinstrument, umfassend eine Steuerungsvorrichtung, wie sie hierin beschrieben und/oder beansprucht ist, wobei das elektrochirurgische Instrument dazu eingerichtet ist, dass mittels der Steuerungsvorrichtung zwischen zwei unterschiedlichen, jeweils von einem Generator des elektrochirurgischen Instruments erzeugbaren Bipolarströmen umschaltbar ist. Insbesondere kann durch wenigstens zwei Schaltsignale der Steuerungsvorrichtung wenigstens ein bipolarer Koagulationsstrom und ein bipolarer Versiegelungsstrom aktivierbar sein. Das elektrochirurgische Instrument kann einen Endeffektor aufweisen, der eine Maulgeometrie mit zwei sich in geschlossenem Zustand gegenüberliegenden Branchen aufweist, deren Offen- und Schließstellung über die Stellung der Griffteile steuerbar sind. Die beiden Branchen können zwei zueinander isolierte Elektroden aufweisen, über welche ein HF-Stromfluss über einen dazwischenliegenden Gewebeabschnitt möglich ist. Dadurch kann über die Branchen eine Versiegelung und/oder eine Koagulation und/oder ein Schneiden des Gewebes erfolgen.

Ein HF-Strom im Sinne der Erfindung kann beispielsweise auf eine Frequenz ab 200 kHz, vorzugsweise ab 300 kHz, definiert sein (wie insbesondere durch die Norm 60601-2-2).

Gemäß einer bevorzugten Ausgestaltung kann es vorgesehen sein, dass eine Aktivierungsdauer für einen Koagulationsstrom und/oder einem Schneidestrom theoretisch unbegrenzt ist und/oder durch die Dauer der Betätigung des wenigstens einen Schaltelements definiert ist. Somit kann ein Nutzer anwendungs- und/oder situationsabhängig entscheiden, wie lange er die Aktivierung aufrechterhält. Vorzugsweise erfolgt somit keine automatisierte Abschaltung des Koagulationsstroms und/oder des Schneidestroms.

Alternativ oder ergänzend dazu kann gemäß einer weiteren bevorzugten Ausgestaltung vorgesehen sein, dass eine Aktivierungsdauer für einen Versiegelungsstrom zeitabhängig geregelt ist. Insbesondere erfolgt eine automatische Abschaltung unabhängig davon, ob eine Betätigung des beweglichen Griffteils und/oder des Steuerelements nach Erreichen einer maximalen Aktivierungsdauer erfolgt. Hierbei kann insbesondere eine Voreinstellung einer gewebespezifischen maximalen Aktivierungsdauer hinterlegt oder hinterlegbar sein. Weiter kann vorzugsweise generatorseitig eine Impedanzmessung erfolgen, wobei abhängig von der gemessenen Impedanz eine Deaktivierung des Versiegelungsstroms automatisiert erfolgt.

Nach der Aktivierung erfolgt eine automatische Abschaltung des Versiegelungsstroms nach Erreichen einer maximalen Aktivierungsdauer, die beispielsweise voreingestellt und/oder impedanzabhängig festgelegt ist. Somit kann verhindert werden, dass es zu Gewebeschäden aufgrund einer zu langen Aktivierung des Stromflusses kommt. Das automatische Abschalten kann dabei beispielsweise durch ein automatisches Abschalten eines Schaltsignals, insbesondere auf Seiten der Steuervorrichtung, und/oder generatorseitig erfolgen. Eine maximale Aktivierungsdauer kann zum Beispiel minimal eine Sekunde und/oder maximal 15 Sekunden, vorzugsweise minimal 2 Sekunden und/oder maximal 10 Sekunden, betragen.

Gemäß einer vorteilhaften Weiterbildung kann es somit vorgesehen sein, dass das elektrochirurgische Instrument zwei gegeneinander isolierte Elektroden aufweist, zwischen denen eine Spannung, insbesondere über einen Generator des elektrochirurgischen Instruments erzeugbare Spannung, anlegbar ist, insbesondere wobei die Elektroden jeweils durch freie Enden eines Endeffektors, vorzugsweise einer bipolaren Pinzette und/oder einer bipolaren Klemme und/oder eines Versiegelungsinstruments, ausgebildet sind, wobei die Elektroden dazu eingerichtet sein können, beabstandet von einander an ein Gewebe angesetzt zu werden, so dass über das Gewebe ein Stromkreis geschlossen ist.

Vorzugsweise weist das elektrochirurgische Instrument keine Neutralelektrode auf und/oder ist nicht für eine monopolare Anwendung eingerichtet. Vielmehr kann es vorgesehen sein, dass das elektrochirurgische Instrument ein aktives Elektrodenpaar aufweist, das zwei Aktivelektroden umfasst.

Die Erfindung wird nun anhand mehrerer Ausführungsbeispiele näher beschrieben, ist jedoch nicht auf diese Ausführungsbeispiele beschränkt.

Es zeigen:
- Fig. 1: eine offene Seitenansicht einer möglichen Ausführungsvariante einer erfindungsgemäßen Steuerungsvorrichtung,
- Fig. 2: eine Explosionsdarstellung des beweglichen Griffteils der Steuerungsvorrichtung aus Fig. 1,
- Fig. 3: eine Seitenansicht des zusammengebauten beweglichen Griffteil aus Fig. 2,
- Fig. 4: eine Detailansicht des als Schaltwippe ausgestalteten Aktivierungselements der Ausführungsvariante der Steuervorrichtung aus Fig. 1, wobei hier ein Status eines haptischen Feedbacks vor einer automatischen Aktivierung gezeigt ist,
- Fig. 5: eine weitere Detailansicht des Aktivierungselements aus Fig. 4, wobei das Aktivierungselement im Status einer automatischen Aktivierung vorliegt,
- Fig. 6: eine weitere Detailansicht des Aktivierungselements aus den Fig. 4 und 5, wobei das Aktivierungselement im deaktivierten Status und/oder vor einem Ausfahren des Hebels vorliegt.

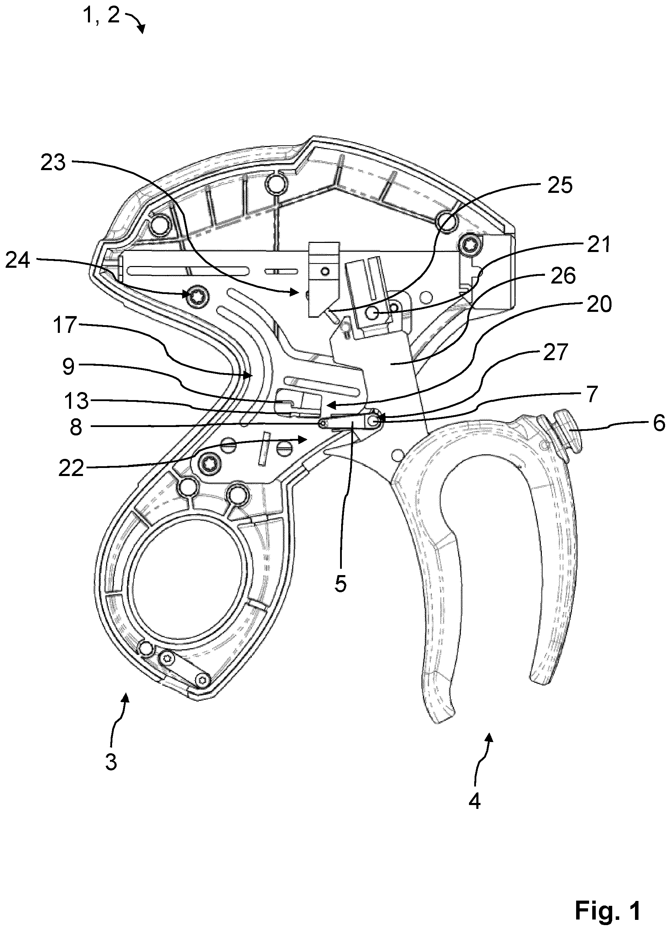

Fig. 1 zeigt eine mögliche Ausgestaltung einer erfindungsgemäßen Steuerungsvorrichtung 1 für ein elektrochirurgisches Instrument 2.

Das elektrochirurgische Instrument 2 kann zum Beispiel ein Hochfrequenz-Instrument zur Erzeugung mehrerer unterschiedlicher Bipolarströme sein.

Die Steuerungsvorrichtung 1 weist einen Handgriff mit einem feststehenden Griffteil 3 und einem insbesondere über ein Drehgelenk beweglichen Griffteil 4 auf. Der Handgriff kann durch einen Nutzer in einer Hand gehalten und gleichzeitig mit dieser Hand eine Verstellung des beweglichen Griffteils 4 relativ zum feststehenden Griffteil 3 vorgenommen werden.

Das bewegliche Griffteil 4 steht in einer Wirkverbindung, insbesondere einer mechanischen Wirkverbindung, mit einem Aktivierungselement 5. Das Aktivierungselement 5 ist dazu eingerichtet, einen Stromkreis zu schließen und damit einen Fluss eines Versiegelungsstroms zu aktivieren. Dies geschieht durch eine Betätigung des beweglichen Griffteils 4 aus einer Deaktivierungsstellung in wenigstens eine Aktivierungsstellung, beispielsweise in einen Aktivierungsbereich mit mehreren Aktivierungstellungen.

Zur Ausbildung des Stromkreises hat die Steuerungsvorrichtung 1 einen ersten Stromanschluss 23 und einen zweiten gegenpoligen Stromanschluss 24. An die Stromanschlüsse 23 und 24 kann eine Stromquelle, insbesondere eine Hochfrequenz-Stromquelle, angeschlossen werden. Der Stromanschluss 24 ist an dem leitend ausgebildeten Trägerelement 17 ausgebildet. Er kann wie eingezeichnet beispielsweise an einem Befestigungsmittel ausgebildet sein oder auch an einer anderen Stelle des Trägerelements 17 oder eines anderen leitenden Bauteils der Steuerungsvorrichtung. Der Stromanschluss 23 ist wie eingezeichnet an einem leitenden Kontaktpin 25 ausgebildet, er kann allerdings auch an einem anderen leitenden Bauteil der Steuerungsvorrichtung ausgebildet sein.

Der Kontaktpin 25 ist von dem Trägerelement 17 isoliert, sodass kein unmittelbarer Stromfluss zwischen diesen ausgebildet werden kann. Ein Strom kann vielmehr von dem Stromanschluss 23 über den Kontaktpin 25, der in einer teilweise angezogenen Stellung des beweglichen Griffteils 4 einen in dem proximalen Element 26 des Griffteils 4 ausgebildeten elektrischen Leiter kontaktiert, und sodann über diesen Leiter zu dem Aktivierungselement 5 fließen. Hierzu kontaktiert der Leiter das Aktivierungselement 5 an einem Kontaktelement 27. Das Kontaktelement 27 kann eine leitende Oberfläche des Aktivierungselements 5 oder auch ein leitender Zapfen oder dergleichen sein. Der Strom fließt sodann durch das Aktivierungselement 5 zu dessen Kontaktelement 8, welches bei noch weiter angezogenem Griffteil 4 die Gegenkontaktfläche 9 kontaktiert. Die Gegenkontaktfläche 9 ist elektrisch mit dem Trägerelement 17 verbunden, sodass ein geschlossener Stromkreis für den Versiegelungsstrom ausgebildet werden kann.

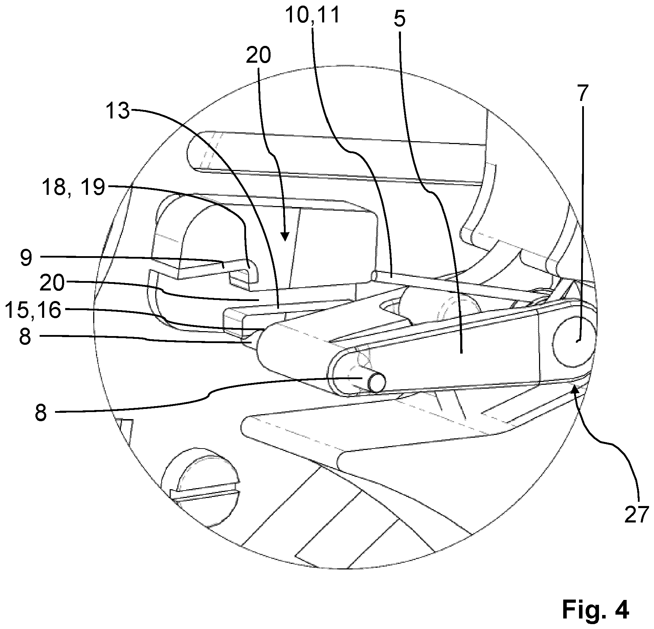

In den Fig. 4-6 ist eine beispielhafte Ausgestaltung eines Aktivierungselements 5 als Schaltwippe gezeigt, wobei das Aktivierungselement 5 hier zur Aktivierung eines Versiegelungsstroms vorgesehen ist. Das Aktivierungselement 5 kann somit um eine Drehachse 7 bei einer Betätigung des beweglichen Griffteils 4 verstellt werden.

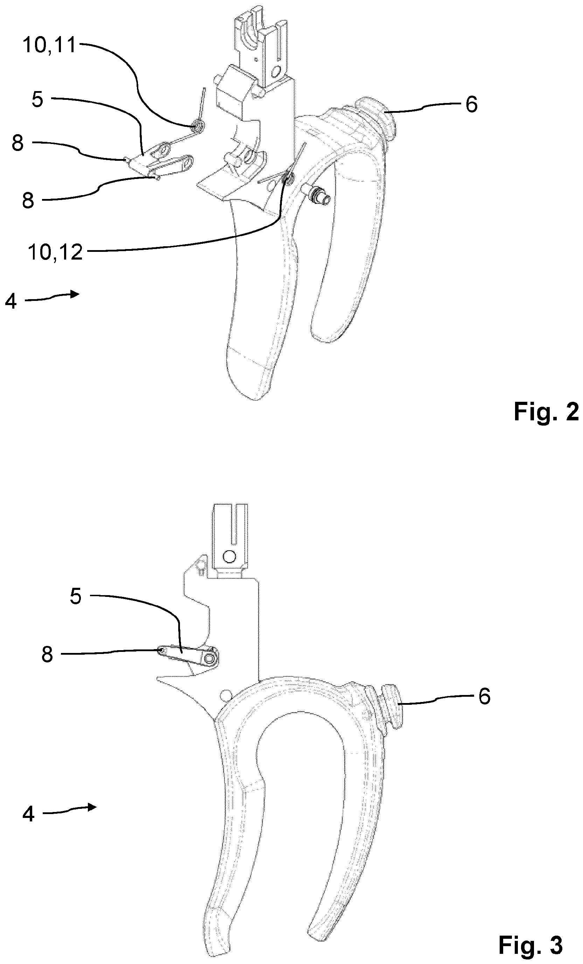

Die Steuervorrichtung 1 umfasst weiter wenigstens ein Schaltelement 6 zum Aktiveren eines Koagulationsstroms und/oder eines Schneidestroms. Wie in den Fig. 1, 2 und 3 zu sehen ist, ist das Schaltelement 6 als ein Druckknopf am beweglichen Griffteil 4 ausgebildet. Ein Nutzer kann somit mit nur einer Hand gleichzeitig einen Versiegelungsstrom und einen Koagulationsstrom und/oder einen Schneidestrom bevorzugt unabhängig voneinander aktivieren. Für den Koagulationsstrom können die gleichen Stromanschlüsse 23 und 24 verwendet werden, die auch für den Versiegelungsstrom verwendet werden. Beispielsweise kann der in dem proximalen Element 26 des Griffteils 4 ausgebildete Leiter eine Bifurkation aufweisen, wobei die zweite Leitung zu dem Schaltelement 6 geführt wird. Das Schaltelement 6 kann sodann mit dem Trägerelement 17 elektrisch in Kontakt stehen.

Die Aktivierung des Schaltelements 6 erfolgt somit durch Drücken, wobei sie unabhängig von einer Betätigung des beweglichen Griffteils 4 möglich ist.

Durch die Betätigung des beweglichen Griffteils 4 und/oder die Betätigung des Schaltelements 6 ist jeweils ein spezifisches Schaltsignal an einen Generator des elektrochirurgischen Instruments 2 aktivierbar, wobei in Abhängigkeit des Schaltsignals ein bestimmter Typ eines Hochfrequenz (HF)-Stroms, insbesondere einer von wenigstens zwei unterschiedlichen Bipolarströmen, zum Beispiel wie zuvor genannt, erzeugt wird.

Wie in den Fig. 1, 2 und 3 gezeigt ist, ist das Aktivierungselement 5 über seine Drehachse 7 am beweglichen Griffteil 4 angeordnet.

Am Aktivierungselement 5 ist beispielsweise an einem freien Ende ein Kontaktelement 8 mit einer leitenden Kontaktfläche ausgebildet. Das feststehende Griffteil 3 und/oder ein Trägerelement 17 weisen/weist ein Gegenkontaktelement 9 mit einer leitenden Gegenkontaktfläche auf.

In einer Aktivierungsstellung des Aktivierungselements 5 kontaktieren sich die leitendenden Kontaktfläche und die Gegenkontaktfläche. Diese Kontaktierung bleibt bei einer weiteren Verstellung des beweglichen Griffteils 4 relativ zum feststehenden Griffteil 3 über einen Aktivierungsabschnitt, also einem Teilabschnitt eines Gesamtverstellweges, bestehen, so dass ein Stromkreis geschlossen ist. Der Strom fließt hierbei durch das Aktivierungselement 5 hindurch. Das Kontaktelement 8 wird entlang des Aktivierungsabschnitts gleitend entlang der Gegenkontaktfläche 9 geführt.

Das Aktivierungselement 5 steht in einer Wirkverbindung mit mindestens einem Rückstellelement 10, insbesondere mit zwei, vorzugsweise entgegen einander wirkenden Rückstellelementen 10. Bei einer Betätigung des beweglichen Griffteils 4 wird das Aktivierungselement 5 entgegen einer Rückstellkraft der Rückstellelemente 10 aus einer Ruheposition ausgelenkt. In der Ruheposition wirkt keine Kraft der sich gegenseitig neutralisierenden Krafteinwirkungen der Rückstellelemente 10 auf das Aktivierungselement 5. Sobald das bewegliche Griffteil 4 betätigt wird, muss das Aktvierungselement 5 entgegen der Rückstellkraft wenigstens eines Rückstellelements 10 verstellt werden. Die Rückstellelemente 10 können beispielsweise als ein erstes Federelement 11 und ein zweites Federelement 12, insbesondere als Schenkelfedern, ausgebildet sein.

Damit ein Nutzer zumindest haptisch erkennen kann, wann es zu einer Kontaktierung zwischen dem Kontaktelement 8 und der Gegenkontaktfläche 9 kommt, weist die Steuerungsvorrichtung 1 wenigstens ein Feedbackelement 15 auf. Mittels des Feedbackelements 15 kann dem Nutzer bei einer Bewegung des beweglichen Griffteils 4 eine haptische Rückmeldung vermittelt werden, wenn eine Verstellung des Aktivierungselements 5 zwischen einer Deaktivierungsstellung und einer Aktivierungsstellung erfolgt. Das Feedbackelement 15 kann beispielsweise als eine Mulde 16 in einer Führungsfläche 14 eines Führungselements 13 ausgebildet sein.

In Fig. 4 ist gezeigt, dass das als ein Pin ausgestaltete Kontaktelement 8 durch die Betätigung des beweglichen Griffteils 4 entlang der Führungsfläche 14 des Führungselements 13 bis zum als Mulde 16 ausgestalteten Feedbackelement 15 geführt wurde und das Kontaktelement 8 in die Mulde 16 eintaucht. Um das Kontaktelement 8 aus der Mulde 16 herauszubewegen, muss der Nutzer einen wahrnehmbaren Widerstand durch Aufbringen einer leicht erhöhten Betätigungskraft überwinden. Durch das haptische Feedback weiß der Nutzer, dass er bei einer weiteren Betätigung eine Aktivierung des Versiegelungsstroms vornimmt, indem das Kontaktelement 8 aus dem Feedbackelement 15 zum Gegenkontaktelement 9 bewegt wird.

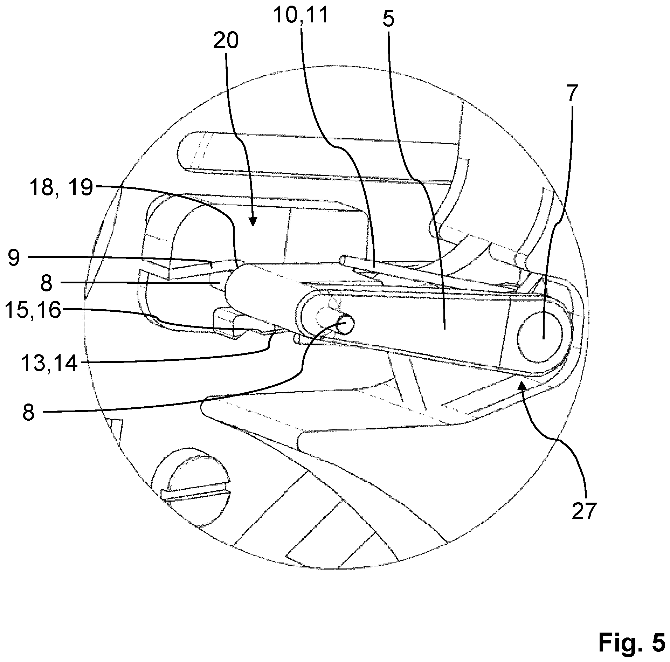

Fig 5 zeigt das Kontaktelement 8 in Aktivierungsstellung, wie es an der Gegenkontaktfläche des Gegenkontaktelements 9 anliegt, wodurch der Stromkreis geschlossen ist. Das Kontaktelement 8 wird bei einer weiteren Betätigung des beweglichen Griffteils 4 entlang der Gegenkontaktfläche des Gegenkontaktelements 9 geführt, wobei der Stromkreis über diesen Aktivierungsabschnitt geschlossen bleibt.

Eine Deaktivierung eines Versiegelungsstroms kann zeitabhängig durch eine Regelung des Generators des elektrochirurgischen Instruments 2 erfolgen. Besonders vorteilhaft ist es, wenn eine Impedanzmessung vorgenommen wird, wobei eine zeitabhängige Deaktivierung des Versiegelungsstroms am Generator erfolgt, wenn ein bestimmter Impedanzwert erreicht wird, insbesondere über- oder unterschritten wird.

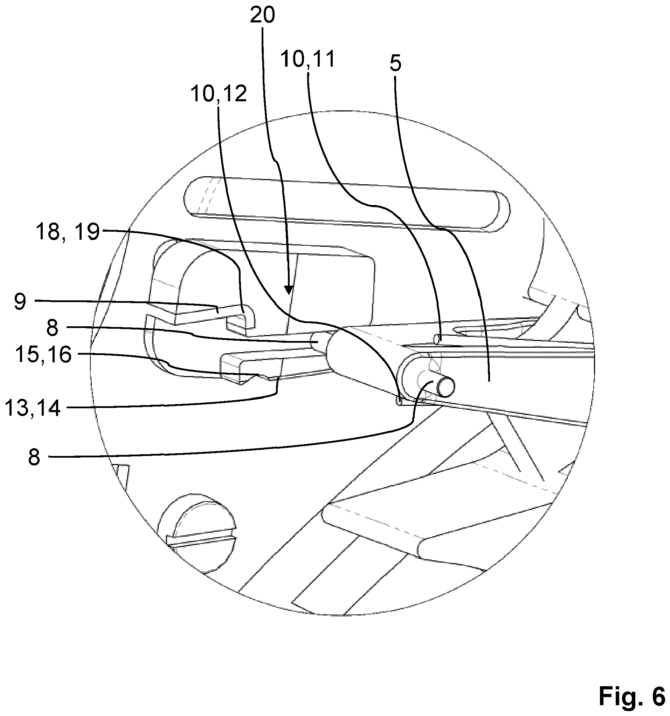

Eine Deaktivierung des Versiegelungsstroms kann außerdem durch ein Zurückführen des beweglichen Griffteils 4 in seine Ruhelage erreicht werden, indem das Kontaktelement 8 dabei vom Gegenkontaktelement 9 entfernt wird, so dass der Stromkreis geöffnet ist. Das Zurückstellen des beweglichen Griffteils 4 aus der Aktivierungsposition in die Deaktivierungsposition ist in Fig. 6 gezeigt. Hier liegt somit keine Kontaktierung zwischen dem Kontaktelement 8 und dem Gegenkontaktelement 9 vor.

Das Gegenkontaktelement 9 kann, wie in den Fig. 4-6 zu sehen ist, zum Beispiel als ein Kontaktblech ausgebildet sein.

### Bezugszeichenliste

- 1: Steuerungsvorrichtung für ein elektrochirurgisches Instrument
- 2: Elektrochirurgisches Instrument
- 3: Feststehendes Griffteil
- 4: Bewegliches Griffteil
- 5: Aktivierungselement (hier z.B. Schaltwippe)
- 6: Schaltelement
- 7: Drehachse des Aktivierungselements
- 8: Kontaktelement des Aktivierungselements
- 9: Gegenkontaktelement (hier z.B. Kontaktblech)
- 10: Rückstellelement
- 11: Erstes Federelement
- 12: Zweites Federelement
- 13: Führungselement
- 14: Führungsfläche
- 15: Feedbackelement
- 16: Mulde
- 17: Trägerelement
- 18: Richtungsänderung
- 19: Kurve
- 20: Kulissenführung
- 21: Drehachse des beweglichen Griffteils
- 22: Sicherheitseinrichtung
- 23: Stromanschluss
- 24: Weiterer Stromanschluss
- 25: Kontaktpin
- 26: Element von 4
- 27: Kontaktelement

## Patentansprüche

1. Steuerungsvorrichtung (1) für ein elektrochirurgisches Instrument (2), insbesondere für ein Hochfrequenz-Versiegelungsinstrument, umfassend ein feststehendes Griffteil (3), ein bewegliches Griffteil (4), ein Aktivierungselement (5) zum Aktivieren eines Stromflusses, wobei das Aktivierungselement (5) bei einer Betätigung des beweglichen Griffteils (4) aus einer Deaktivierungsstellung in wenigstens eine Aktivierungsstellung verstellt wird und wobei bei einer Betätigung des beweglichen Griffteils (4) das Aktivierungselement (5) um einen Drehpunkt verstellt wird, **dadurch gekennzeichnet, dass** das Aktivierungselement so ausgebildet ist, dass ein durch das Aktivierungselement aktivierter Strom durch das Aktivierungselement fließt oder fließen kann.

2. Steuerungsvorrichtung (1) für ein elektrochirurgisches Instrument (2), insbesondere nach dem Oberbegriff aus Anspruch 1 oder nach Anspruch 1, umfassend wenigstens ein feststehendes Griffteil (3), ein bewegliches Griffteil (4), wenigstens ein Schaltelement (6) zum Aktivieren eines Stromflusses, insbesondere eines zweiten Typs, vorzugsweise eines Koagulationsstroms und/oder eines Schneidestroms, **dadurch gekennzeichnet, dass** das wenigstens eine Schaltelement (6) am beweglichen Griffteil (4) ausgebildet ist, wobei das wenigstens eine Schaltelement (6) durch einen Benutzer unabhängig von einer Betätigung des beweglichen Griffteils (4) betätigbar ist.

3. Steuerungsvorrichtung (1) für ein elektrochirurgisches Instrument (2), insbesondere nach dem Oberbegriff aus Anspruch 1 oder nach einem der Ansprüche 1 oder 2, umfassend wenigstens ein Schaltelement (6), insbesondere durch einen Nutzer unmittelbar betätigbares und/oder sprachsteuerbares Schaltelement (6), zum Aktivieren und/oder Umschalten eines bipolaren Stromflusses, **dadurch gekennzeichnet, dass** durch das wenigstens ein Schaltelement (6) ein Aktivieren und/oder ein Umschalten zwischen wenigstens zwei unterschiedlichen Schaltsignalen möglich ist, um durch einen Generator des elektrochirurgischen Instruments (2) in Abhängigkeit vom jeweiligen Schaltsignal wenigstens zwei unterschiedliche Bipolarströme zu erzeugen, insbesondere wobei durch die wenigstens zwei Schaltsignale wenigstens ein bipolarer Koagulationsstrom und/oder wenigstens ein bipolarer Schneidestrom und ein bipolarer Versiegelungsstrom aktivierbar ist.

4. Steuerungsvorrichtung (1) nach einem der vorstehenden Ansprüche, **dadurch gekennzeichnet, dass** ein oder das Aktivierungselement (5) am beweglichen Griffteil (4) oder am feststehenden Griffteil (3) ausgebildet oder angeordnet ist.

5. Steuerungsvorrichtung (1) nach einem der vorstehenden Ansprüche, **dadurch gekennzeichnet, dass** das Aktivierungselement (5) wenigstens ein leitendes Kontaktelement (8, 27) aufweist und dass die Steuerungsvorrichtung (1) eine leitende Gegenkontaktfläche (9) aufweist, die in einer Aktivierungsstellung durch das Kontaktelement (8, 27) beaufschlagt ist.

6. Steuerungsvorrichtung (1) nach dem vorangehenden Anspruch, **dadurch gekennzeichnet, dass** das Kontaktelement (8, 27) bei einer Verstellung des beweglichen Griffteils (4) über einen Aktivierungsabschnitt eines Gesamtverstellweges des beweglichen Griffteils (4) und/oder solange das Aktivierungselement (5) in einer Aktivierungsstellung ist, entlang der Gegenkontaktfläche (9) gleitend geführt ist.

7. Steuerungsvorrichtung (1) nach einem der vorstehenden Ansprüche, **dadurch gekennzeichnet, dass** die Steuerungsvorrichtung (1) wenigstens ein Rückstellelement (10) aufweist, insbesondere zwei, vorzugsweise entgegen einander wirkende, Rückstellelemente (10) aufweist, wobei bei einer Betätigung des beweglichen Griffteils (4) das Aktivierungselement (5) entgegen einer Rückstellkraft des wenigstens einen Rückstellelements (10) aus einer Ruheposition auslenkbar ist oder ausgelenkt wird, vorzugsweise wobei in der Ruheposition keine Krafteinwirkung des wenigstens einen Rückstellelements (10) oder sich gegenseitig neutralisierende Krafteinwirkungen der Rückstellelemente (10) auf das Aktivierungselement (5) wirken.

8. Steuerungsvorrichtung (1) nach einem der vorstehenden Ansprüche, **dadurch gekennzeichnet, dass** die Steuerungsvorrichtung (1) wenigstens ein Feedbackelement (15) aufweist, mit welchem dem Nutzer bei einer Bewegung des beweglichen Griffteils (4) eine haptische und/oder akustische Rückmeldung vermittelbar ist, insbesondere wenn eine Verstellung des Aktivierungselements (5) zwischen einer Deaktivierungsstellung und einer Aktivierungsstellung erfolgt.

9. Steuerungsvorrichtung (1) nach einem der vorstehenden Ansprüche, **dadurch gekennzeichnet, dass** wenigstens ein oder das wenigstens eine Schaltelement (6) an dem beweglichen Griffteil (4) und/oder an dem feststehenden Griffteil (3) und/oder an einem Fußschaltelement ausgebildet ist.

10. Elektrochirurgisches Instrument (2), umfassend eine Steuerungsvorrichtung (1) nach einem der vorstehenden Ansprüche, **dadurch gekennzeichnet, dass** das elektrochirurgische Instrument (2) dazu eingerichtet ist, dass mittels der Steuerungsvorrichtung (1) zwischen zwei unterschiedlichen, jeweils von einem Generator des elektrochirurgischen Instruments (2) erzeugbaren Bipolarströmen umschaltbar ist, insbesondere wobei durch wenigstens zwei Schaltsignale der Steuerungsvorrichtung (1) wenigstens ein bipolarer Koagulationsstrom und ein bipolarer Versiegelungsstroms aktivierbar ist.

## Claims

1. Control device (1) for an electrosurgical instrument (2), in particular for a high-frequency sealing instrument, comprising a fixed handle part (3), a movable handle part (4), an activation element (5) for activating a current flow, wherein the activation element (5) is adjusted from a deactivation position into at least one activation position when the movable handle part (4) is actuated, and wherein the activation element (5) is adjusted about a pivot point when the movable handle part (4) is actuated, **characterized in that** the activation element is designed such that a current activated by the activation element flows or can flow through the activation element.

2. Control device (1) for an electrosurgical instrument (2), in particular according to the preamble of Claim 1 or according to Claim 1, comprising at least a fixed handle part (3), a movable handle part (4), at least one switching element (6) for activating a current flow, in particular of a second type, preferably a coagulation current and/or a cutting current, **characterized in that** the at least one switching element (6) is formed on the movable handle part (4), wherein the at least one switching element (6) can be actuated by a user independently of an actuation of the movable handle part (4).

3. Control device (1) for an electrosurgical instrument (2), in particular according to the preamble of Claim 1 or according to either of Claims 1 and 2, comprising at least one switching element (6), in particular a switching element (6) that can be directly operated and/or voice-controlled by a user, for activating and/or switching a bipolar current flow, **characterized in that** the at least one switching element (6) enables an activation and/or a switchover between at least two different switching signals, in order to generate at least two different bipolar currents by a generator of the electrosurgical instrument (2) depending on the respective switching signal, in particular wherein at least one bipolar coagulation current and/or at least one bipolar cutting current and a bipolar sealing current can be activated by the at least two switching signals.

4. Control device (1) according to one of the preceding claims, **characterized in that** an or the activation element (5) is formed or arranged on the movable handle part (4) or on the fixed handle part (3).

5. Control device (1) according to one of the preceding claims, **characterized in that** the activation element (5) has at least one conductive contact element (8, 27), and **in that** the control device (1) has a conductive mating contact surface (9) which, in an activation position, is acted upon by the contact element (8, 27).

6. Control device (1) according to the preceding claim, **characterized in that**, during an adjustment of the movable handle part (4) over an activation section of a total adjustment path of the movable handle part (4) and/or as long as the activation element (5) is in an activation position, the contact element (8, 27) is guided slidingly along the mating contact surface (9).

7. Control device (1) according to one of the preceding claims, **characterized in that** the control device (1) has at least one reset element (10), in particular two reset elements (10) preferably acting in opposition to each other, wherein, when the movable handle part (4) is actuated, the activation element (5) is deflectable or deflected from a rest position counter to a restoring force of the at least one reset element (10), preferably wherein, in the rest position, no force of the at least one reset element (10) acts on the activation element (5) or mutually neutralizing forces of the reset elements (10) act on the activation element (5).

8. Control device (1) according to one of the preceding claims, **characterized in that** the control device (1) has at least one feedback element (15) with which the user can be given a haptic and/or acoustic feedback when the movable handle part (4) is moved, in particular when the activation element (5) is adjusted between a deactivation position and an activation position.

9. Control device (1) according to one of the preceding claims, **characterized in that** at least one or the at least one switching element (6) is formed on the movable handle part (4) and/or on the fixed handle part (3) and/or on a foot-operated switch element.

10. Electrosurgical instrument (2), comprising a control device (1) according to one of the preceding claims, **characterized in that** the electrosurgical instrument (2) is configured to be switchable, by means of the control device (1), between two different bipolar currents that can each be generated by a generator of the electrosurgical instrument (2), in particular wherein at least one bipolar coagulation current and a bipolar sealing current can be activated by at least two switching signals of the control device (1).

## Revendications

1. Dispositif de commande (1) pour un instrument électro-chirurgical (2), en particulier pour un instrument de clampage à haute fréquence, comprenant une poignée fixe (3), une poignée mobile (4), un élément d'activation (5) pour activer un flux de courant, dans lequel l'élément d'activation (5) passe d'une position de désactivation à au moins une position d'activation en actionnant la poignée mobile (4) et dans lequel l'élément d'activation (4) est déplacé autour d'un point de rotation en actionnant la poignée mobile (4), **caractérisé en ce que** l'élément d'activation (5) est conçu de sorte qu'un courant activé par l'élément d'activation circule ou peut circuler à travers l'élément d'activation.

2. Dispositif de commande (1) pour un instrument électro-chirurgical (2), en particulier selon le préambule de la revendication 1 ou selon la revendication 1, comprenant au moins une poignée fixe (3), une poignée mobile (4), au moins un élément de commutation (6) pour activer un flux de courant, en particulier d'un deuxième type, de préférence un courant de coagulation et/ou un courant de coupe, **caractérisé en ce que** l'au moins un élément de commutation (6) est formé sur la poignée mobile (4), l'au moins un élément de commutation (6) pouvant être actionné par un utilisateur indépendamment d'un actionnement de la poignée mobile (4).

3. Dispositif de commande (1) pour un instrument électro-chirurgical (2), en particulier selon le préambule de la revendication 1 ou selon une des revendications 1 ou 2, comprenant au moins un élément de commutation (6), en particulier en particulier un élément de commutation (6) actionnable directement et/ou contrôlable vocalement par un utilisateur, pour activer et/ou commuter un flux de courant bipolaire, **caractérisé en ce qu'**il est possible avec l'au moins un élément de commutation (6) d'activer et/ou d'alterner entre au moins deux signaux de commutation différents pour générer au moins deux courants bipolaires différents avec un générateur de l'instrument électro-chirurgical (2) en fonction du signal de commutation, en particulier les au moins deux signaux de commutation permettant d'activer au moins un courant de coagulation bipolaire et/ou au moins un courant de coupe bipolaire et un courant de clampage bipolaire.

4. Dispositif de commande (1) selon une des revendications précédentes, **caractérisé en ce qu'**un ou l'élément d'activation (5) est configuré ou agencé sur la poignée mobile (4) ou sur la poignée fixe (3).

5. Dispositif de commande (1) selon une des revendications précédentes, **caractérisé en ce que** l'élément d'activation (5) comporte au moins un élément de contact conducteur (8, 27) et que ce dispositif de commande (1) comporte une surface de contact opposée conductrice (9) qui est touchée par l'élément de contact (8, 27) dans une position d'activation.

6. Dispositif de commande (1) selon la revendication précédente, **caractérisé en ce que** l'élément de contact (8, 27) est guidé de façon à coulisser le long de la surface de contact opposée (9) lors d'un déplacement de la poignée mobile (4) sur une section d'activation du chemin de déplacement total de la poignée mobile (4) et/ou tant que l'élément d'activation (5) est dans une position d'activation.

7. Dispositif de commande (1) selon une des revendications précédentes, **caractérisé en ce que** ce dispositif de commande (1) comporte au moins un élément de rappel (10), en particulier deux éléments de rappel (10) agissant de préférence en sens contraires, l'élément d'activation (5) pouvant être sorti ou étant sorti, lors d'un actionnement de la poignée mobile (4), d'une position de repos en s'opposant à une force de rappel de l'au moins un élément de rappel (10), de préférence aucune force de l'au moins un élément de rappel (10) ou aucunes forces se neutralisant mutuellement des éléments de rappel (10) n'agissant sur l'élément d'activation (5) en position de repos.

8. Dispositif de commande (1) selon une des revendications précédentes, **caractérisé en ce que** ce dispositif de commande (1) comporte au moins un élément de réaction (15) avec lequel une réaction haptique et/ou acoustique peut être transmise à l'utilisateur lors d'un mouvement de la poignée mobile (4), en particulier lorsqu'un déplacement de l'élément d'activation (5) entre une position de désactivation et une position d'activation a lieu.

9. Dispositif de commande (1) selon une des revendications précédentes, **caractérisé en ce qu'**au moins un ou l'au moins un élément de commutation (6) est formé sur la poignée mobile (4) et/ou sur la poignée fixe (3) et/ou sur n élément de commutation à pied.

10. Instrument électro-chirurgical (2) comprenant un dispositif de commande (1) selon une des revendications précédentes, **caractérisé en ce que** cet instrument électro-chirurgical (2) est équipé pour pouvoir alterner entre deux courants bipolaires différents pouvant être générés chacun par un générateur de l'instrument électro-chirurgical (2), en particulier au moins un courant de coagulation bipolaire et un courant de clampage bipolaire pouvant être activés par au moins deux signaux de commutation du dispositif de commande (1).
